# EUROPÄISCHE PATENTSCHRIFT

(11) **EP 0 846 676 B1**
(45) Veröffentlichungstag und Bekanntmachung des Hinweises auf die Patenterteilung: **07.02.2001**
(21) Anmeldenummer: 97120611.5
(22) Anmeldetag: 25.11.1997
(51) Int. Cl.: C07C 209/10

(54) **Synthese von aromatischen Aminen aus Chloraromaten**
Process for the preparation of aromatic amines from aryl chlorides
Procédé de préparation d'amines aromatiques à partir de chlorures aromatiques

(30) Priorität: 04.12.1996 DE 19650213
(43) Veröffentlichungstag der Anmeldung: 10.06.1998
(73) Patentinhaber: Axiva GmbH, 65926 Frankfurt am Main (DE)
(72) Erfinder: Beller, Matthias, Prof Dr., 85737 Ismaning (DE); Riermeier, Thomas, 80939 München (DE); Reisinger, Claus Peter, DI., 85748 Graiching (DE); Herrmann, Wolfgang Anton, Prof. Dr., 85354 Freising (DE); Geissler, Holger, Dr., 55116 Mainz (DE)

(56) Entgegenhaltungen:
- US-A- 5 576 460
- LOUIE J ET AL: "Palladium-Catalyzed Synthesis of Arylamines from Aryl Halides Mechanistic Studies Lead to Coupling in the Absence of Tin Reagents" TETRAHEDRON LETTERS, Bd. 36, Nr. 21, 22.Mai 1995, Seite 3609-3612 XP004028031
- NAGAVELLI PRABHAKAR REDDY ET AL.: "Palladium-catalyzed amination of aryl chlorides" TETRAHEDRON LETTERS., Bd. 38, Nr. 27, 1997, OXFORD GB, Seiten 4807-4810, XP002056351

## Beschreibung

Die vorliegende Erfindung betrifft ein Verfahren zur Herstellung von aromatischen Aminen aus den entsprechenden Chloraromaten, insbesondere ein Verfahren zur Herstellung von substituierten Anilinen, unter Verwendung eines Palladiumkatalysators.

Aromatische Amine, speziell substituierte Aniline, sind von großer industrieller Bedeutung als Vorprodukte für Farbstoffe, Feinchemikalien, Pharmazeutika und Agroprodukte.

Die Herstellung von substituierten Anilinen wird technisch i.a. durch Nitrierung eines entsprechenden Aromaten und nachfolgende Hydrierung durchgeführt. Da Nitrierung unter drastischen Reaktionsbedingungen stattfinden, sind eine Vielzahl komplexer substituierter Aniline nicht oder nur unzureichend auf diesem Wege darstellbar.

Alternative Herstellwege für Aniline sind die Ammonolyse von Phenolen und Chlorbenzol (K. Weissermel, J.-J. Arpe, Industrielle Organische Chemie, 3. Auflage, S. 396 ff.). Aufgrund der Verfahrensweise, wie der erforderlichen drastischen Reaktionsbedingungen (Temperaturen > 180-420°C; Drucke 60-200 bar), sind auch diese Reaktionen nicht für die Herstellung von substituierten Anilinen geeignet.

Kürzlich wurden neue palladiumkatalysierte Aminierungen von Jod- und Bromaromaten, die zu substituierten Anilinen führen, in A.S. Guram, R.A. Rennels, S.L. Buchwald, Angew. Chem. 1995, 107, 1456 und J. Louie, J.F. Hartwig, Tetrahedron Lett., 1995, 36, 3609 beschrieben. Diese Reaktionen werden unter vergleichsweise milden Reaktionsbedingungen durchgeführt und können daher auch für unterschiedlich substituierte Aniline eingesetzt werden. Die als Ausgangsverbindungen eingesetzten Iod- und Bromaromaten sind jedoch teuer und nur schwer zugänglich.

Aus US-A-5576460 ist ein Verfahren gemäß dem Oberbegriff des Anspruches 1 bekannt, bei dem jedoch die Verwendung von Chloraromaten zusammen mit einem Halogenid-Cokatalysator nicht beschrieben wird.

Daher ist es von großem industriellen Interesse, ein Verfahren zu entwickeln, mit dem substituierte aromatische Amine, insbesondere auch komplexe substituierte Aniline, aus allgemein verfügbaren Ausgangsverbindungen, die zudem billig sind, unter milden Reaktionsbedingungen hergestellt werden

Erfindungsgemäß wird diese Aufgabe gelöst durch ein Verfahren gemäß Anspruch 1.

Mit dem erfindungsgemäßen Verfahren lassen sich beispielsweise Verbindungen wie Arylpiperazine, Aryldibutylamine, Arylphenylmethylamine, Aryldiethylamine, Arylphenylamine, wobei Aryl bevorzugt für Phenyl, Methoxyphenyl, Trifluormethylphenyl, Acetylphenyl, Fluorphenyl, Difluorphenyl, Methylphenyl, Pyridyl und Naphthyl steht, auf einfache Weise erhalten.

Für das erfindungsgemäße Verfahren enthält der Palladiumkatalysator vorzugsweise mindestens einen phosphorhaltigen Liganden.

Der Palladiumkatalysator kann bereits den mindestens einen phosphorhaltigen Liganden enthalten, der phosphorhaltige Liganden enthaltende Palladiumkatalysator kann aber auch aus Katalysatorvorstufen erzeugt werden.

Als Katalysatorvorstufen werden dabei vorzugsweise Palladacyclen und/oder Palladium-(O)- oder -(II)-verbindungen in Gegenwart von phosphorhaltigen Liganden wie Phosphanliganden eingesetzt.

Geeignete Beispiele für Palladacyclen sind trans-Di(µ-aceto)-bis[o(di-o-tolylphosphino)benzyl]dipalladium II sowie die entsprechenden brom- oder chlorverbrückten Verbindungen oder Verbindungen, in denen der Tolylrest durch Mesityl- oder t-Butylreste ersetzt sein kann.

Als Palladiumverbindungen können Pd(0)-Komplexverbindungen und Pd(II)-Verbindungen verwendet werden. Geeignete Beispiele sind Palladiumacetate, -halogenide, -nitrate, -carbonate, -ketonate, -acetylacetonate, Nitrilpalladiumhalogenide, Olefinpalladiumhalogenide, Allylpalladiumhalogenide sowie Palladiumbiscarboxylate.
Konkrete Vertreter sind z.B. Pd(OAc)₂, Pd(acac)₂, (CH₃CN)₂Pd(NO₂)Cl, (C₁₀H₈N₂)PdCl₂, Pd₂(dba)₃ und PdCl₂.

Die Palladiumverbindung kann auch in situ erzeugt werden, beispielsweise aus Palladium-(II)-acetat oder Palladium-(II)-chlorid durch Zugabe eines üblichen Reduktionsmittels.

Vorzugsweise handelt es sich bei den phosphorhaltigen Liganden um ein- oder zweizähnige Phosphanliganden.

Als Phosphanliganden geeignete Verbindungen sind beispielsweise Triphenylphosphan, Tricyclohexylphosphan, Bisdiphenylphosphinoethan, Bisdiphenylphosphinopropan, Bisdiphenylphosphinobutan, Tri-n-butylphosphan, Triisopropylphosphan, Bisdiphenylphosphinobenzol, Bisdiphenylphosphinobinaphthyl, Diphenylphosphinopyridin, wobei die Phenylreste substituiert oder gegebenenfalls durch ein oder mehrere C₁ bis C₁₂-Alkylgruppen oder C₃ bis C₁₀-Cycloalkylgruppen ersetzt sein können. Ein besonders bevorzugter Ligand ist Tri-o-tolylphosphan.

Die Liganden werden i.a. in einem P/Pd-Verhältnis von 8:1 bis 1:1 eingesetzt. Der Katalysator wird im allgemeinen in Mengen von 0,0001 mol-% bis 10 mol-%, bevorzugt 0,001 mol-% bis 5 mol-% (bezogen auf den Chloraromaten) eingesetzt.

Amine reagieren nach dem erfindungsgemäßen Verfahren mit Chloraromaten palladiumkatalysiert in Gegenwart einer starken Base, deren pKₐ-Wert vorzugsweise größer 10 ist. Als Basen sind beispielsweise stark basische Alkali- und Erdalkaliderivate wie Alkali- und Erdalkalialkoholate, Alkali- und Erdalkaliamide sowie Butyllithium, Phenyllithium etc. verwendbar. Bevorzugte Basen sind Alkali- und Erdalkalialkoholate wie Natrium-tert.-butanolat, Kalium-tert.-butanolat, Lithium-tert.-butanolat, Natriumphenolat, Kaliumphenolat und Kaliumcarbonat, Natriumhexamethyldisilazid sowie Lithiumhexamethyldisilazid.

Die Base wird vorzugsweise in einer Menge von 0,5 - 5 Äquivalenten, insbesondere von 1 - 3 Äquivalenten und ganz besonders bevorzugt von 1,2 - 2 Äquivalenten, bezogen auf den Chloraromaten, eingesetzt.

Als Lösungsmittel dienen üblicherweise inerte organische Lösungsmittel. Bevorzugt sind aromatische Kohlenwasserstoffe wie Toluol, Xylole, Anisol, Tetralin und aliphatische Ether wie Tetrahydrofuran, Dimethoxyethan, Dioxan, Tetrahydropropan, Formaldehydacetale.

Die Umsetzung der Chloraromaten findet bei Temperaturen von 80 bis 200°C, bevorzugt bei und besonders bevorzugt bei 120 - 120 - 180°C 180°C statt.

Als Halogenid-Cokatalysator dienen Alkali- und/oder Erdalkalibromide, -chloride oder -iodide. Auch Ammonium- und Phosphoniumbromide, -chloride oder -iodide können verwendet werden. Bevorzugte Halogenide sind die entsprechenden Bromide wie Lithiumbromid, Natriumbromid, Kaliumbromid und Tetraalkylammoniumbromide. Der Halogenid-Co-Katalysator wird in Mengen von 0,1 - 100 mol-%, bevorzugt von 3 - 50 mol-%, bezogen auf den Chloraromaten, eingesetzt. Er kann auch als flüssiges Salz als Lösungsmittel dienen.

Das erfindungsgemäße Verfahren ist besonders geeignet für die Umsetzung von primären aromatischen Aminen und sekundären aliphatischen und aromatischen Aminen. Üblicherweise wird das Amin, bezogen auf den Chloraromaten, in annähernd stöchiometrischen Mengen oder im Überschuß zugesetzt. Vorzugsweise beträgt die Menge Amin 1 bis 3 Äquivalente, insbesondere 1,2 bis 2 Äquivalente.

Besonders günstig für Umsetzungen wirken sich auch elektronenziehende Substituenten am Chloraromaten aus.
Beispiele für geeignete elektronenziehende Substituenten sind NO₂, R-(CO)-, CF₃-, F, CN, wobei R ein verzweigter oder linearer Alkylrest sein kann.

Die Umsetzung erfolgt vorzugsweise unter einem üblichen Schutzgas wie N₂, He oder Ar.

Die substituierten Arylamine bilden sich nach dem erfindungsgemäßen Verfahren generell in guten bis sehr guten Ausbeuten von 50 - 99 %. Als Nebenprodukte werden vereinzelt Dehalogenierungsprodukte beobachtet, die leicht abtrennbar sind.

In Erweiterung der intermolekularen Kupplung können auch einfache intramolekulare Aminierungen durchgeführt werden.

Ausgangsverbindungen dafür können 2-Arylethylamine, 3-Arylpropylamine, 4-Arylbutylamine, 2-Arylpropylamine sein, wobei Aryl die vorstehend genannte Bedeutung hat.

Die nachfolgenden Beispiele dienen lediglich zur Erläuterung des Verfahren ohne es darauf zu beschränken.

### Beispiele:

Allgemeine Arbeitsvorschrift (AAV): 20 mmol Chloraromat, 24 mmol Amin, 28 mmol KOtBu, 4 mmol Lithiumbromid und 1 mol-% Palladiumkatalysator werden in 100 ml Toluol suspendiert und unter Schutzgas in ein Druckrohr gegeben und in einem, auf 140°C erwärmten, Ölbad gerührt. Dabei beobachtet man eine Braunfärbung des gelben Reaktionsansatzes. Nach 24 h läßt man auf Raumtemperatur abkühlen, filtriert von den Salzen ab und wäscht mit Diethylether nach. Die Lösemittel werden am Rotationsverdampfer entfernt. Durch Säulenchromatographie (Hexan/Essigester: 50/1) erhält man das gewünschte Produkt.

### Beispiel 1

Nach AAV werden 2,0 mmol 4-Chlorbenzotrifluorid, 2,4 mmol Piperidin, 2,8 mmol Kalium-tert.-butylat, 0,4 mmol Lithiumbromid, 1,0 mol-% (9,4 mg) Palladacyclus (trans-Di(µ-aceto)-bis[o[di-o-tolylphosphino)benzyl]dipalladium II) in 10 ml Toluol umgesetzt. Man erhält 4-Trifluormethyl-phenylpiperidin und 3-Trifluormethylphenylpiperidin im Verhältnis 6:1 in 65 % Ausbeute.

### Beispiel 2

Nach AAV werden 2,0 mmol 4-Chlorbenzotrifluorid, 2,4 mmol Morpholin, 2,8 mmol Kalium-tert.-butylat, 0,4 mmol Lithiumbromid, 1,0 mol-% (9,4 mg) Palladacyclus (trans-Di(µ-aceto)-bis[o[di-o-tolylphosphino)benzyl]dipalladium II) in 10 ml Toluol umgesetzt. Man erhält 4-Trifluormethyl-phenylmorpholin und 3-Trifluormethylphenylmorpholin im Verhältnis 5:1 in 62 % Ausbeute.

### Beispiel 3

Nach AAV werden 2,0 mmol 4-Chlorbenzotrifluorid, 2,4 mmol Di-n-butylamin, 2,8 mmol Kalium-tert.-butylat, 0,4 mmol Lithiumbromid, 1,0 mol-% (9,4 mg) Palladacyclus (trans-Di(µ-aceto)-bis[o[di-o-tolylphosphino)benzyl]dipalladium II) in 10 ml Toluol umgesetzt. Man erhält 4-Trifluormethylphenyl-di-n-butylamin und 3-Trifluormethylphenyl-di-n-butylamin im Verhältnis 7:1 in 58 % Ausbeute.

### Beispiel 4

Nach AAV werden 2,0 mmol 4-Chlorfluorbenzol, 2,4 mmol Piperidin, 2,8 mmol Kalium-tert.-butylat, 0,4 mmol Lithiumbromid, 1,0 mol-% (9,4 mg) Palladacyclus (trans-Di(µ-aceto)-bis[o[di-o-tolylphosphino)benzyl]dipalladium II) in 10 ml Toluol umgesetzt. Man erhält 4-Fluorphenylpiperidin und 3-Fluorphenylpiperidin im Verhältnis 2:1 in 75 % Ausbeute.

## Patentansprüche

1. Verfahren zur Herstellung von aromatischen Aminen der allgemeinen Formel (I)
Ar-NR¹R² (I)
worin Ar für 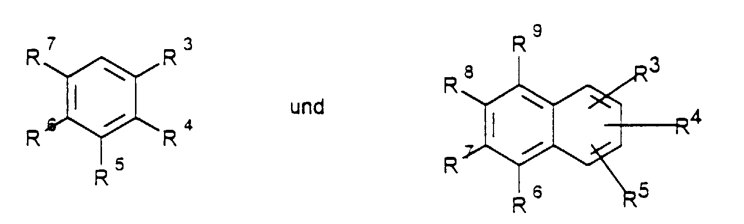 steht und R³ bis R⁹ unabhängig voneinander Wasserstoff, C₁-C₈-Alkyl, Alkoxy-(C₁-C₈), Acyloxy-(C₁-C₈), OAr, Ar, Fluor, Chlor, Brom, lod, OH, NO₂, CN, CO₂H, CHO, SO₃H, SO₂R, SOR, NH₂, NHAlkyl-(C₁-C₁₂), NAlkyl₂-(C₁-C₁₂), NHCO-Alkyl-(C₁-C₁₂), CO-Alkyl-(C₁-C₁₂), NHCHO, COAr, CO₂Ar, CF₃, CONH₂, CHCHCO₂R, POAr₂, POAlkyl₂-(C₁-C₁₂),
Pyridyl , SiAlkyl₃-(C₁-C₁₂) mit R = Alkyl-(C₁-C₈), Aryl-(C₅-C₁₀) bedeuten und R¹ und R² unabhängig voneinander für Wasserstoff, C₁-C₁₂-Alkyl, Ar mit der genannten Bedeutung,
Pyridyl stehen, durch Umsetzung von Chloraromaten der Formel II
Ar-Cl (II)
mit Ammoniak oder primären oder sekundären Aminen der Formel III
R¹R²NH (III)
worin Ar und R¹ und R² die angegebene Bedeutung besitzen, in Gegenwart eines inerten Lösungsmittels, eines Palladiumkatalysators und einer Base deren pKₐ-Wert größer als 10 ist und bei Temperaturen von 80 - 200°C, **dadurch gekennzeichnet**, daß man einem Alkali-, Erdalkali-, Ammonium- oder Phosphonium-Halogenid als Cokatalysator zusetzt.

2. Verfahren gemäß Anspruch 1, wobei R1 und R2 unabhängig voneinander Wasserstoff, C1-C12-Alkyl oder Ar
worin Ar für 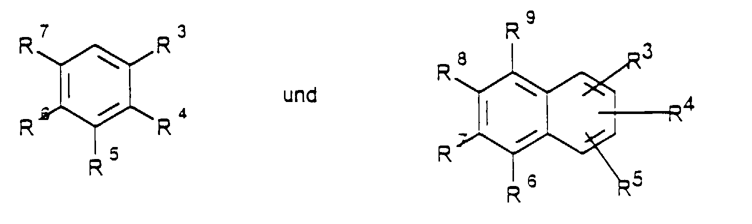 steht und R³ bis R⁹ unabhängig voneinander Wasserstoff, C₁-C₈-Alkyl, Alkoxy-(C₁-C₈), Acyloxy-(C₁-C₈), OAr, Ar, Fluor, Chlor, Brom, lod, OH, NO₂, CN, CO₂H, CHO, SO₃H, SO₂R, SOR, NH₂, NHAlkyl-(C₁-C₁₂), NAlkyl₂-(C₁-C₁₂), NHCO-Alkyl-(C₁-C₁₂), CO-Alkyl-(C₁-C₁₂), NHCHO, COAr, CO₂Ar, CF₃, CONH₂, CHCHCO₂R, POAr₂, POAlkyl₂-(C₁-C₁₂), Pyridyl , SiAlkyl₃-(C₁-C₁₂) mit R = Alkyl-(C₁-C₈), Aryl-(C₅-C₁₀) bedeuten und R¹ und R² unabhängig voneinander für Wasserstoff, C₁-C₁₂-Alkyl, Ar mit der genannten Bedeutung, stehen, umsetzt.

3. Verfahren gemäß Anspruch 1, wobei der Palladiumkatalysator mindestens einen phosphorhaltigen Liganden enthält.

4. Verfahren gemäß Anspruch 3, wobei der mindestens eine phosphorhaltige Ligand ausgewählt ist unter Triphenylphosphan, Tricyclohexylphosphan, Bisdiphenylphosphinoethan, Bisdiphenylphosphinopropan, Bisdiphenylphosphinobutan, Tri-n-butylphosphan, Triisopropylphosphan, Bisdiphenylphosphinobenzol, Bisdiphenylphosphinobinaphthyl, Diphenylphosphinopyridin und Tri-o-tolylphosphan.

5. Verfahren nach einem der Ansprüche 1 bis 4, wobei der Palladiumkatalysator in situ aus einer Palladiumverbindung und mindestens einem phosphorhaltigen Liganden erzeugt wird.

6. Verfahren nach Anspruch 5, wobei die Palladiumverbindung ausgewählt ist unter Palladacyclen, Pd(0)-Komplexverbindungen und Pd(II)-verbindungen.

7. Verfahren nach Anspruch 6, wobei die Palladiumverbindung ausgewählt ist unter Pd(OAc)₂, Pd(aca)₂, (CH₃CN)₂Pd(NO₂)Cl, (C₁₀H₈N₂)PdCl₂, Pd(dba)₃ und PdCl₂.

8. Verfahren nach einem der Ansprüche 1 bis 7, wobei die Base eine Alkali- und/oder Erdalkalibase ist.

9. Verfahren nach Anspruch 8, wobei die starke Base ausgewählt ist unter Alkali- und Erdalkalialkoholate, Alkali- und Erdalkaliamide, Butyllithium, Phenyllithium, Kaliumcarbonat, Natriumhexamethyldisilazid und Lithiumhexamethyldisilazid.

10. Verfahren zur Herstellung von Mischungen aus 4-Trifluormethyl-phenylpiperidin und 3- Trifluormethyl-phenylpiperidin durch Umsetzung von 4-Chlorbenzotrifluorid und Piperidin in Toluol in Gegenwart von Kalium-tert- butylat und des Palladacyclus (trans-Di-(µ-aceto)-bis[o[di-o-tolylphosphino]benzyl]dipalladium-II) bei 140°C, dadurch gekennzeichnet, daß Lithiumbromid als Cokatalysator zugesetzt wird.

11. Verfahren zur Herstellung von Mischungen aus 4-Trifluormethyl-phenylmorpholin und 3- Trifluormethyl-phenylmorpholin durch Umsetzung von 4-Chlorbenzotrifluorid und Morpholin in Toluol in Gegenwart von Kalium-tert- butylat und des Palladacyclus (trans-Di-(µ-aceto)-bis[o[di-o-tolylphosphino]benzyl]dipalladium-II) bei 140°C, dadurch gekennzeichnet, daß Lithiumbromid als Cokatalysator zugesetzt wird.

12. Verfahren zur Herstellung von Mischungen aus 4-Fluorphenylpiperidin und 3-Fluorphenylpiperidin durch Umsetzung von 4-Chlorfluorbenzol und Piperidin in Toluol in Gegenwart von Kalium-tert-butylat und des Palladacyclus (trans-Di-(µ-aceto)-bis[o[di-o-tolylphosphino]benzyl]dipalladium-II) bei 140°C, dadurch gekennzeichnet, daß Lithiumbromid als Cokatalysator zugesetzt wird.

## Claims

1. A process for the preparation of aromatic amines of the formula (I)
Ar-NR¹R² (I)
in which Ar is 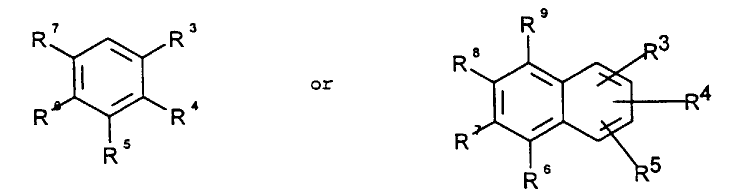 and R³ to R⁹ independently of one another are hydrogen, C₁-C₈-alkyl, alkoxy-(C₁-C₈), acyloxy-(C₁-C₈), OAr, Ar, fluorine, chlorine, bromine, iodine, OH, NO₂, CN, CO₂H, CHO, SO₃H, SO₂R, SOR, NH₂, NH-alkyl-(C₁-C₁₂), N-alkyl₂-(C₁-C₁₂), NHCO-alkyl-(C₁-C₁₂), CO-alkyl-(C₁-C₁₂), NHCHO, COAr, CO₂Ar, CF₃, CONH₂, CHCHCO₂R, POAr₂, PO-alkyl₂-(C₁-C₁₂), pyridyl, Si-alkyl₃-(C₁-C₁₂) where R = alkyl(C₁-C₈), aryl-(C₅-C₁₀) and R¹ and R² independently of one another are hydrogen, C₁-C₁₂-alkyl, Ar as defined above, pyridyl, by reacting chloroaromatics of the formula II
Ar-Cl (II)
with ammonia or primary or secondary amines of the formula III
R¹R²NH (III)
in which Ar and R¹ and R² are as defined above, in the presence of an inert solvent, a palladium catalyst and a base whose pKₐ value is greater than 10 and at temperatures of 80-200°C wherein an alkali metal halide, alkaline earth metal halide, ammonium halide or phosphonium halide is added as cocatalyst.

2. The process as claimed in claim 1, wherein R1 and R2, independently of one another, are hydrogen, C1-C12-alkyl or Ar
in which Ar is 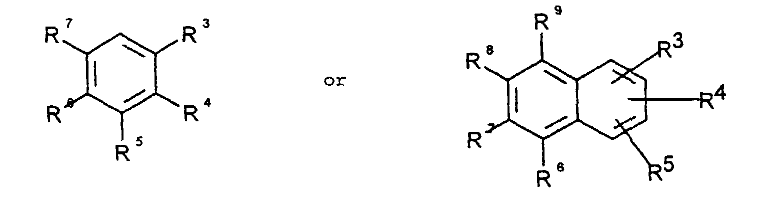 and R3 to R9, independently of one another, are hydrogen, C₁-C₈-alkyl, alkoxy-(C₁-C₈), acyloxy-(C₁-C₈), OAr, Ar, fluorine, chlorine, bromine, iodine, OH, NO₁, CN, CO₂H, CHO, SO₃H, SO₂R, SOR, NH₂, NH-alkyl-(C₁-C₁₂), N-alkyl₂-(C₁-C₁₂), NHCO-alkyl-(C₁-C₁₂), CO-alkyl-(C₁-C₁₂), NHCHO, COAr, CO₂Ar, CF₃, CONH₂, CHCHCO₂R, POAr₂, PO-alkyl₂-(C₁-C₁₂), pyridyl, Si-alkyl₃-(C₁-C₁₂) where R = alkyl(C₁-C₈), aryl-(C₅-C₁₀) and R¹ and R² independently of one another are hydrogen, C₁-C₁₂-alkyl, Ar as defined above.

3. The process as claimed in claim 1, wherein the palladium catalyst contains at least one phosphorus-containing ligand.

4. The process as claimed in claim 3, wherein the phosphorus-containing ligand is chosen from triphenylphosphine, tricyclohexylphosphine, bisdiphenylphosphinoethane, bisdiphenylphosphinopropane, bisdiphenylphosphinobutane, tri-n-butylphosphine, triisopropylphosphine, bisdiphenylphosphinobenzene, bisdiphenylphosphinobinaphthyl, diphenylphosphinopyridine and tri-o-tolylphosphine.

5. The process as claimed in one of claims 1 to 4, wherein the palladium catalyst is prepared in situ from a palladium compound and at least one phosphorus-containing ligand.

6. The process as claimed in claim 5, wherein the palladium compound is chosen from palladacycles, Pd(0) complex compounds and Pd(II) compounds.

7. The process as claimed in claim 6, wherein the palladium compound is chosen from Pd(OAc)₂, Pd(aca)₂, (CH₃CN)₂Pd(NO₂)Cl, (C₁₀H₈N₂)PdCl₂, Pd(dba)₃ and PdCl₂.

8. The process as claimed in one of claims 1 to 7, wherein the base is an alkali metal base and/or alkaline earth metal base.

9. The process as claimed in claim 8, wherein the strong base is chosen from alkali metal and alkaline earth metal alkoxides, alkali metal and alkaline earth metal amides, butyllithium, phenyllithium, potassium carbonate, sodium hexamethyldisilazide and lithium hexamethyldisilazide.

10. A process for the preparation of mixtures of 4-trifluoromethylphenylpiperidine and 3-trifluoromethylphenylpiperidine by reacting 4-chlorobenzotrifluoride and piperidine in toluene in the presence of potassium tert-butoxide and the palladacycle trans-di(p-aceto)bis[o[di-o-tolylphosphino]benzyl]dipalladium(II) at 14°C, wherein lithium bromide is added as cocatalyst.

11. A process for the preparation of mixtures of 4-trifluoromethylphenylmorpholine and 3-trifluoromethylphenylmorpholine by reacting 4-chlorobenzotrifluoride and morpholine in toluene in the presence of potassium tert-butoxide and the palladacycle trans-di(µ-aceto)bis[o[di-o-tolylphosphino]benzyl]dipalladium(II) at 14°C, wherein lithium bromide is added as cocatalyst.

12. A process for the preparation of mixtures of 4-fluorophenylpiperidine and 3-fluorophenylpiperidine by reacting 4-chlorofluorobenzene and piperidine in toluene in the presence of potassium tert-butoxide and the palladacycle trans-di(p-aceto)bis[o[di-o-tolylphosphino]benzyl]dipalladium(II) at 14°C, wherein lithium bromide is added as cocatalyst

## Revendications

1. Procédé pour la préparation d'amines aromatiques de formule générale (I)
Ar-NR¹R² (I)
dans laquelle Ar représente 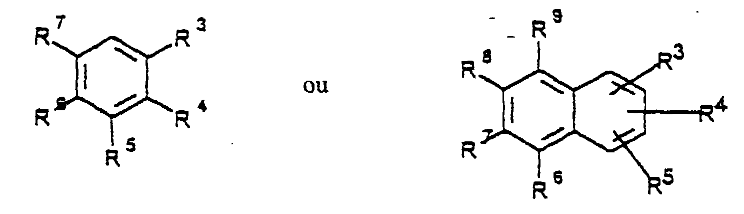 et R³ à R⁹ représentent indépendamment les uns des autres un atome d'hydrogène, un groupe alkyle en C₁ à C₈, alcoxy en C₁ à C₈, acyloxy en C₁ à C₈, OAr, Ar, fluor, chlore, brome, iode, OH, NO₂, CN, CO₂H, CHO, SO₃H, SO₂R, SOR, NH₂, NH-alkyle en C₁ à C₁₂, N-(alkyle en C₁ à C₁₂)₂ NHCO-alkyle en C₁ à C₁₂, CO-alkyle en C₁ à C₁₂, NHCHO, COAr, CO₂Ar, CF₃, CONH₂, CHCHCO₂R, POAr₂, PO(alkyle en C₁ à C₁₂)₂, pyridyle, Si-(alkyle en C₁ à C₁₂)₃ avec R = alkyle en C₁ à C₈, aryle en C₅ à C₁₀ et R¹ et R² représentent indépendamment l'un de l'autre un atome d'hydrogène, un groupe alkyle en C₁ à C₁₂, Ar avec la signification indiquée, pyridyle, consistant à mettre à réagir des composés aromatiques chlorés de formule II
Ar-Cl (II)
avec l'ammoniac ou des amines primaires ou secondaires de formule III
R¹R²NH (III)
dans lesquelles Ar et R¹ et R² possèdent la signification indiquée,
en présence d'un solvant inerte, d'un catalyseur au palladium et d'une base dont la valeur du pKₐ est supérieure à 10 et à des températures variant de 80 à 200°C, **caractérisé en ce que**, l'on ajoute comme co-catalyseur un halogénure de métal alcalin, alcalino-terreux, d'ammonium ou de phosphonium.

2. Procédé selon la revendication 1, dans lequel R¹ et R² représentent indépendamment l'un de l'autre un atome d'hydrogène, un groupe alkyle en C₁ à C₁₂ ou Ar dans lequel Ar représente 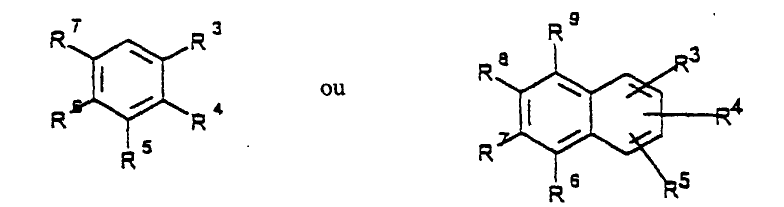 et R³ à R⁹ représentent indépendamment les uns des autres un atome d'hydrogène, un groupe alkyle en Ci à C₈, alcoxy en C₁ à C₈, acyloxy en C₁ à C₈, OAr, Ar, fluor, chlore, brome, iode, OH, NO₂, CN, CO₂H, CHO, SO₃H, SO₂R, SOR, NH₂, NH-alkyle en C₁ à C₁₂, N-(alkyle en C₁ à C₁₂)₂ NHCO-alkyle en C₁ à C₁₂, CO-alkyle en C₁ à C₁₂, NHCHO, COAr, CO₂Ar, CF₃, CONH₂, CHCHCO₂R, POAr₂, PO(alkyle en C₁ à C₁₂)₂, pyridyle, Si-(alkyle en C₁ à C₁₂)₃ avec R = alkyle en C₁ à C₈, aryle en C₅ à C₁₀ et R¹ et R² représentent indépendamment l'un de l'autre un atome d'hydrogène, un groupe alkyle en C₁ à C₁₂, Ar avec la signification indiquée.

3. Procédé selon la revendication 1, dans lequel le catalyseur au palladium contient au moins un ligand phosphoré.

4. Procédé selon la revendication 3, dans lequel au moins un ligand phosphoré est choisi parmi le triphénylphosphane, le tricyclohexylphosphane, le bisdiphénylphosphinoéthane, le bisdiphénylphosphinopropane, le bisdiphénylphosphinobutane, le tri-n-butylphosphane, le triisopropylphosphane, le bisdiphénylphosphinobenzène, le bisdiphénylphosphinobinatphtyle, la diphénylphosphinopyridine et le tri-o-tolylphosphane.

5. Procédé selon l'une des revendications 1 à 4, dans lequel le catalyseur au palladium est produit in situ à partir d'un composé de palladium et d'au moins un ligand phosphoré.

6. Procédé selon la revendication 5, dans lequel le catalyseur au palladium est choisi parmi les composés cycliques du palladium, les composés complexes de Pd(0) et les composés du Pd(II).

7. Procédé selon la revendication 6, dans lequel le composés de Pd est choisi parmi Pd(OAC)₂, Pd(aca)₂, (CH₃CN)₂Pd(NO₂)Cl, (C₁₀H₈N₂)PdCl₂, Pd₂(dba)₃ et PdCl₂.

8. Procédé selon l'une des revendications 1 à 7, dans lequel la base est une base de métaux alcalins ou alcalino-terreux.

9. Procédé selon la revendication 8, dans lequel la base forte est choisie parmi les alcoolates de métaux alcalins et alcalino-terreux, les amides de métaux alcalins et alcalino-terreux, le butyllithium, le phényllithium, le carbonate de potassium, l'hexaméthyldisilazoture de sodium et l'hexaméthyldisilazoture de lithium.

10. Procédé pour la préparation de mélanges de 4-trifluorométhyl-phénylpipéridine et de 3-trifluorométhyl-phénylpipéridine en mettant à réagir le trifluorure de 4-chlorobenzène et la pipéridine dans le toluène en présence de tert.-butylate de potassium et du composé cyclique du palladium trans-di(µ-acéto)-bis [o(di-o-tolylphosphino)benzyl]dipalladium II à 140°C, caractérisé en ce que, le bromure de lithium est ajouté comme co-catalyseur.

11. Procédé pour la préparation de mélanges de 4-trifluorométhyl-phénylmorpholine et de 3-trifluorométhyl-phénylmorpholine en mettant à réagir le trifluorure de 4-chlorobenzène et la morpholine dans le toluène en présence de tert.-butylate de potassium et du composé cyclique du palladium trans-di (µ-acéto)-bis [o(di-o-tolylphosphino)benzyl]dipalladium II à 140°C, caractérisé en ce que, le bromure de lithium est ajouté comme co-catalyseur.

12. Procédé pour la préparation de mélanges de 4-fluorophénylpipéridine et de 3-fluorophénylpipéridine en mettant à réagir le 4-chlorofluorobenzène et la pipéridine dans le toluène en présence de tert.-butylate de potassium et du composé cyclique du palladium trans-di(µ-acéto) -bis [o(di-o-tolylphosphino)benzyl]dipalladium II à 140°C, caractérisé en ce que, le bromure de lithium est ajouté comme co-catalyseur.
